# EUROPEAN PATENT SPECIFICATION

(11) **EP 1 591 449 B1**
(45) Date of publication and mention of the grant of the patent: **13.06.2007**
(21) Application number: 05103219.1
(22) Date of filing: 21.04.2005
(51) Int. Cl.: C07K 1/04, C07K 1/06

(54) **A process for the formation of disulfide bonds in cyclic peptides**
Verfahren zur Herstellung von Disulfidbindungen bei zyklischen Peptiden
Procédé pour la formation de liens disulphide chez les peptides cycliques

(30) Priority: 26.04.2004 IT MI20040812
(43) Date of publication of application: 02.11.2005
(73) Proprietor: CHEMI S.p.A., 20092 Cinisello Balsamo (Milano) (IT)
(72) Inventor: Cappelletti, Silvana, 20030 Lentate sul Seveso (MI) (IT); Starace, Olivia, I-20146 Milano (IT); Annoni, Paola, 20092 Cinisello Balsamo (MI) (IT); Pinori, Massimo, 23877 Paderno d'Adda (Lecco) (IT)
(74) Representative: Pistolesi, Roberto

(56) References cited:
- BIOCHEMISTRY., vol. 15, no. 18, 1976, pages 4071-4076, XP002338008 US AMERICAN CHEMICAL SOCIETY. EASTON, PA.
- INTERNATIONAL JOURNAL OF PEPTIDE AND PROTEIN RESEARCH., vol. 45, 1995, pages 152-156, XP000520078 DK MUNKSGAARD, COPENHAGEN.
- DATABASE CA [Online] CHEMICAL ABSTRACTS SERVICE, COLUMBUS, OHIO, US; DANHO, W. ET AL: "Syntheses and biological properties of hybrids between human insulin and insulin-like growth factor I" XP002342361 retrieved from STN Database accession no. 97:110370 & PEPT.: SYNTH., STRUCT., FUNCT., PROC. AM. PEPT. SYMP., 7TH , 113-22. EDITOR(S): RICH, DANIEL H.; GROSS, ERHARD. PUBLISHER: PIERCE CHEM. CO., ROCKFORD, ILL. CODEN: 47LMAO, 1981,

## Description

The present invention relates to a novel method for the formation of an intramolecular disulfide bond in peptide or peptidomimetic molecules.

### Prior art

Many biologically active peptides which are useful in the treatment of various disorders are known to contain disulfide bonds (1, 2). Many of these are already registered as pharmaceuticals, while others are still at the development stage; some significant examples of peptides with disulfide bonds are listed below:

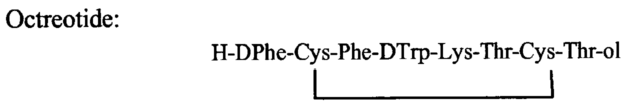

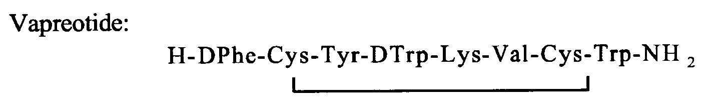

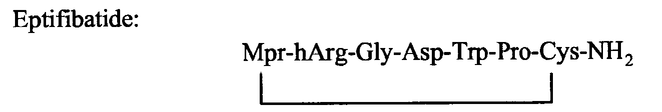

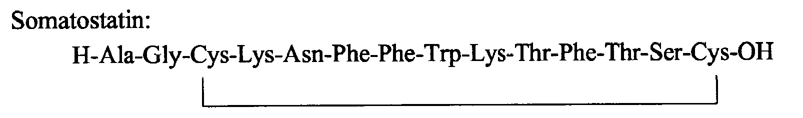

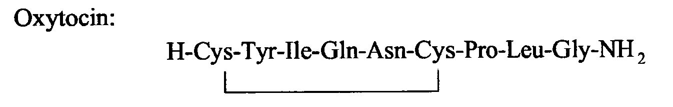

Methods which are conventionally used for the formation of disulfide bonds make use of linear precursors which contain cysteine or other derivatives containing - SH groups in the free form, which are subjected to oxidative processes by means of various oxidising agents. These oxidative methods have been thoroughly described in the literature (3, 4).

The main disadvantage of oxidative methods is the exposure of the peptide molecule to oxidising agents which can bring about the formation of impurities, with consequent low process yields and difficulties in purification. These treatments may furthermore result in the partial inactivation of the peptide from the standpoint of biological activity.

Other disadvantages, especially for the purposes of industrial production, arise from the fact that some of these processes, whether in the -SH group deprotection phase or in the oxidation phase, use reactants such as heavy metals, iodine, potassium ferricyanide, etc., which entail appropriate disposal methods.

A non-oxidative process for the cyclisation of peptides has been described in (5). The principle disadvantage of this method, which makes use of the presence of a preformed -S-S-alkyl group, resides in the necessity of appropriately disposing of the toxic gases (alkyl mercaptans) which are released by the reaction.

It is also known (6) that the -S-sulfonate group (Bunte salt) reacts with the -SH groups to form disulfide bonds; moreover, in those cases known in the literature, the -S-sulfonate group has been introduced into peptide molecules by oxidative sulfitolysis of cysteine residues and this strategy cannot be applied to the preparation of cyclic peptides, which requires the simultaneous presence, in the same molecule, of an -S-sulfonate group and a free -SH group.

The possibility of introducing preformed -S-sulfonate groups during the synthesis of peptide molecules has been described (8). We have now found, and this is the subject matter of the present invention, that the second -SH group in free form, which is necessary for the formation of the intramolecular disulfide bond, may be obtained, in presence of the -S-sulfonate group, by selectively removing, under suitable acidic conditions, a protective group of the trityl or at least acid-labile type.

### Description of the invention

The present invention provides a novel synthesis method, which is simple and non-oxidative, for the cyclisation of peptide or peptidomimetic molecules by means of the formation of intramolecular disulfide bonds; said method comprises:
(a) preparation of a linear precursor containing an -S-sulfonate group and a protected -SH group with acid-labile protection;
(b) selective liberation of the -SH group in an anhydrous acid medium and in the presence of suitable carbocation scavengers;
(c) cyclisation to dissolve the partially deprotected linear precursor at a pH of between 5 and 9.

The process of the present invention may be applied to any linear peptide or peptidomimetic compound which contains at least two cysteines or other residues with -SH groups in the side chain. The amino acid derivative containing a first - SH group (normally a cysteine) is initially protected with a protective group which may be removed selectively with regard to the S-sulfonate group; said protective groups and the associated conditions of use are well known in the art and are described, for example, in (7), which is incorporated herein by reference; preferably, they are selected from among 4-methoxytrityl (Mmt), 2,4,6-trimethoxybenzyl (Tmob), 4,4',4"-trimethoxytrityl (TmTr), trityl (Trt), with trityl (Trt) being the particularly preferred protective group. The remaining one of the two SH groups which will then be used to form the disulfide bond is thus protected with the S-sulfonate group; such S-sulfonate groups and the associated application methods are likewise known from the art and are, for example, described in (8), which is incorporated herein by reference.

In practice, the peptide to be cyclised is synthesised chemically using amino acid derivatives containing already protected -SH groups (in the case both of the acid-labile protection and of the sulfonate group), so as to avoid possible selectivity problems.

The subsequent step then involves the selective removal of the first acid-labile protective group; in this manner, a peptide is obtained which comprises a free -SH group and an -SH group protected by an S-sulfonate group. However, known prior art conditions for removing the above-stated protective group exhibit a series of disadvantages; for example, incorrect deblocking of the protective group may damage the polypeptide chain and, in particular, may also deblock the second -SH residue protected by the sulfonate group.

One of the characterising aspects of the present invention is thus that of having found specific reaction conditions which permit selective deblocking of the first - SH group with regard to the -SH group protected by the sulfonate group.

In a preferred aspect of the present invention, selective liberation of the -SH group is performed in an anhydrous aprotic non-polar organic solvent in the presence of anhydrous trifluoroacetic acid and a suitable carbocation scavenger, such as for example triisopropylsilane. Preferably, the protective group is trityl and the deblocking reaction is performed in the presence of a mixture of trifluoroacetic acid and triisopropylsilane in ratios by volume of between 90:10 and 99:1, still more preferably in a ratio of approx. 95:5.

The reaction conditions are compatible with the presence of further scavengers capable of scavenging carbocations, such as for example anisole, derivatives of tryptophan, such as tryptophan methyl ester (H-Trp-OMe) and/or phenol; said scavengers are used in quantities of between 1 and 3, preferably approx. 2, equivalents relative to the peptide to be cyclised; phenol has proved to be the best of these.

Finally, the anhydrous aprotic non-polar solvent is preferably selected from among dichloromethane, chloroform, dichloroethane, trichloroethylene and/or tetrachloroethylene, with dichloromethane being particularly preferred. This solvent is preferably used in a quantity of between 25 and 10 litres, preferably between 20 and 15 litres, per mole of peptide to be cyclised. The preferred ratio between the solvent and trifluoroacetic acid is approx. 2.2:1 by volume.

Simply dissolving the peptide comprising a free -SH group and an -SH group protected by an S-sulfonate group at a pH of between 5 and 9 spontaneously gives rise, without the necessity for other reactants, to the formation of the disulfide bond by displacement of the S-sulfonate group.

As shown in Tables 1 and 2, the percentage conversions over time, reaction times and purity at different pH values were evaluated in order to define the pH range within which to perform the cyclisation. The optimum pH has proved to be between 5 and 9, and in particular the pH used is pH 8.3.

In the Table, 8SH indicates the linear intermediate, while octreotide is the example of a cyclised peptide with a disulfide bridge.

**Table 1**

| | 30' | | 90' | | 150' | |
|---|---|---|---|---|---|---|
| pH | 8SH (HPLC A%) | Octreotide (HPLC A%) | 8SH (HPLC A%) | Octreotide (HPLC A%) | 8SH (HPLC A%) | Octreotide (HPLC A%) |
| pH=3 | 75.9 | 7.4 | 73.8 | 10.3 | 69.9 | 14.5 |
| pH=5. 6 | 15.3 | 71.4 | 4.4 | 78.2 | 4.0 | 77.4 |
| pH=6. 9 | 3.0 | 79.3 | 2.7 | 79.0 | 2.5 | 79.0 |
| pH=8. 3 | 1.7 | 81.5 | 1.5 | 81.3 | 1.4 | 82.1 |
| pH=9. 2 | 1.5 | 84.1 | 0.7 | 84.2 | 0.7 | 84.2 |

**Table 2:**

| pH PH | End of reaction | 8SH (HPLC A%) | Octreotide (HPLC A%) | Other impurities >0.5% (A%) |
|---|---|---|---|---|
| pH=3.0 | 72 h | 3.6 | 76.4 | 13 |
| pH=5.6 | 24 h | 3.5 | 78.6 | 12.7 |
| pH=6.9 | 24 h | 2 | 80.8 | 11 |
| pH=8.3 | 24 h | 1.2 | 81.9 | 10.9 |
| pH=9.2 | 24 h | 0.4 | 88.8 | 7.6 |

Using a pH of less than 5 results in slow reaction times, an incomplete reaction and poor purity. Using a pH of greater than 9 should be avoided due to possible racemisation reactions with the formation of difficult to identify diastereomers, which are typical of peptides with a basic pH (9, 10). Using a pH of greater than 9 should also be avoided due to possible disproportionation reactions which would cause the disulfide bridge to break with the possible formation of dimers or oligomers (11).

Formation of the disulfide bond is thus preferably performed at a pH of between 7 and 9, still more preferably of between 8 and 8.5.

The reaction solvent is preferably a mixture of buffer solution/aprotic polar organic solvent at a suitable pH: the buffer will be prepared using salts such as sodium or potassium phosphates or ammonium acetates, preferably sodium phosphate; the aprotic polar organic solvent will be selected from among acetonitrile, tetrahydrofuran, dimethylformamide, more preferably acetonitrile. Said aprotic polar organic solvent is preferably present in a quantity of 0.5-1.5 volumes per volume of said aqueous buffer, still more preferably in quantity of 0.8-1.2 volumes per volume of said aqueous buffer.

The reaction is moreover preferably performed at a temperature of between 0 and 50°C, still more preferably at a temperature of between 15 and 30°C; the time required to perform cyclisation is usually between 5 minutes and 24 hours, preferably between 5 and 60 minutes.

The resultant peptide may then be isolated and/or subjected to further purification with the methods normally used for the purification of peptides (phase inversion, ion exchange, etc.); in particular, it may be isolated by precipitation, for example by addition of methyl *tert.* -butyl ether (MTBE), ethyl ether, n-hexane or n-heptane to the aqueous buffer, with methyl *tert.* -butyl ether being particularly preferred.

The linear precursor may be prepared in solution or in the solid phase, by sequential attachment of the amino acid derivatives or by fragments, using methods which are well known in peptide synthesis, which provide protection for the side chains, and subsequent protection and deprotection of the terminal amino group. Carboxyl activation makes it possible to use active esters, EDC, DCC or other typical peptide synthesis methods (10).

The novel cyclisation method has been used in the synthesis of various cyclic peptides with a disulfide bond such vapreotide, eptifibatide and octreotide. Scheme 1 shows, purely by way of example, the application of the method to the preparation of the peptide octreotide: the peptide is obtained by cyclisation of the linear precursor H-DPhe-Cys(SO₃Na)-Phe-DTrp-Lys-Thr-Cys-Thr-ol (8SH).

The following Examples are given purely by way of example and do not limit the invention.

### Example 1

The stability of the sulfonate protective group under trityl removal conditions was evaluated, using Fmoc-Cys(SO₃Na)-ONa and Fmoc-Cys(Trt)-OH as model products. The two products (100 mg of each) were suspended separately in dichloromethane (1 ml) and trifluoroacetic acid (0.5 ml) and triisopropylsilane (0.05 ml) were added. The formation of Fmoc-Cys-OH (the product obtained by removal of the protective groups) was evaluated by HPLC.

The formation of Fmoc-Cys-OH was evaluated using a Vydac C18 (5µm) (4.6 × 250)mm column.
Eluent A: H₂O/CH₃CN 90/10 with 0.1% trifluoroacetic acid
Eluent B: CH₃CN with 0.1% trifluoroacetic acid
Gradient: %B: 10-40 (20 minutes), 80 (10 minutes).
Retention time of Fmoc-Cys(SO₃Na)-ONa = 16.9
Retention time of Fmoc-Cys(Trt)-OH = 35.0
Retention time of Fmoc-Cys-OH = 25.3
Fmoc-Cys-OH was identified by means of LC-MS; [M+H]⁺ = 344

| Reaction time (min) | Formation of Fmoc-Cys-OH from Fmoc-Cys(Trt)-OH (A%) | Formation of Fmoc-Cys-OH from Fmoc-Cys(SO₃Na)-ONa (A%) |
|---|---|---|
| 0 | 0.00 | 0.00 |
| 15 | 93.13 | 0.00 |
| 30 | 93.04 | 0.33 |
| 60 | 92.65 | 0.62 |

After 15 minutes, removal of the trityl from Fmoc-Cys(Trt)-OH was practically complete, while no removal of the protective group from Fmoc-Cys(SO₃Na)-ONa was observed, apart from a tiny percentage and that only starting from 30 minutes.

### Example 2

The protected octapeptide [Boc-DPhe-Cys(SO₃Na)-Phe-DTrp-Lys(Boc)-Thr-Cys(Trt)-Thr-ol] (0.27 moles; 420 g) and methylene chloride (4.9 1) were introduced into a 50 litre reactor. Phenol (0.54 moles; 50 g) and trifluoroacetic acid [TFA] (2.19 1), were added and the mixture was stirred for 10 minutes at 23°C. Triisopropylsilane [TIS] (1.2 moles; 247 ml) was added to the solution and the mixture was stirred for 5 minutes. Finally, methyl *tert*.-butyl ether [MTBE] (19.7 1) was added and the mixture stirred for at least 60 minutes. The solid was filtered out, washed with MTBE (10.41) and dried under a vacuum at 30 °C for at least 15 hours.

The solid obtained [2TFA.H-DPhe-Cys(SO₃Na)-Phe-DTrp-Lys-Thr-Cys-Thr-ol] was introduced into a 100 litre reactor together with acetonitrile (39.1 1) and the mixture was stirred at 23°C for at least 5 minutes.

A phosphate buffer solution which had previously been flushed with nitrogen (39.1 1 of 0.2 molar buffer prepared with 1394 g of Na₂HPO₄*2H₂O, 39.1 1 of H₂O and adjusted to pH=8.3 by addition of H₃PO₄) was added to the reaction mixture, and the mixture was stirred under nitrogen at 23°C until the disulfide bond formed. The reaction mixture was acidified with trifluoroacetic acid (0.731 1) to pH =3.

The volatile fraction was removed under a vacuum, then the NaCl (5.7 kg) and tetrahydrofuran (12.6 1) were introduced. The mixture was stirred, allowed to separate out and the phases were separated. The aqueous phase was reintroduced into the reactor with tetrahydrofuran (12.61). The mixture was stirred, allowed to separate out and the phases were separated. The combined organic phases were concentrated under a vacuum until a stirrable residue was obtained. The residue was resuspended three times with isopropanol (29.7 1), then isopropanol (3.5 1) was added. Once the complete removal of the water had been verified by Karl Fischer analysis, the salts were filtered out and washed with isopropanol (1.5 1). The alcoholic solution (8.1 1) was added dropwise to a mixture of n-hexane (60 1) and MTBE (19.91) at 0°C. The precipitate was stirred for at least 15 minutes. The product was filtered out, washed with n-hexane (7.5 1) and dried under a vacuum at 30°C for at least 15 hours.
418 g (0.30 moles) were obtained. LOD: 10%

The product was identified by means of LC-MS: [M+H]⁺ = 1019 and HPLC (identification relative to standard).
HPLC method used:
Column: JUPITER 5µm C18 300A (4.6 × 250 mm)
Eluents: A = 10% ACN + 0.1% TFA; B = ACN + 0.1% TFA
Temperature: 30°C
Flow rate: 1 ml/min
Gradient: %B=10-30(30')-80(10')
Wavelength: 220 nm
Sample: 1 g/l (volume injected: 20 µl)

### Bibliographic references

(1) Handbook of Biochemistry, pages C-164 to C-188
(2) Brazeau, P., et al., Science, 179, 77, 1973
(3) Katsoyannis, P. G., The Chemistry of Polypeptides, Plenum Press, 1974, pages 60-85
(4) Kudryavtseva, E.V, Sidorova, M.V., Ovchinnikov, M.V., Bespalova, Z.D. and Bushuev, V.N., Peptide Res., 49, 1997, 52-58
(5) US-3,929,758, Lawrence et al., 30 December 1975
(6) Schwartz, G. et al., Biochemistry, 15, 1976, 4071-4076
(7) Greene et al., Protective Groups in Organic Synthesis, Third edition, 6th chapter, Wiley Interscience.
(8) Maugras, I., Gosteli, J., Rapp, E., Nyfeler, R., Int. J. Peptide Protein Res., 45, 1995, 152-156
(9) Williams, M.W., Young, G. T., J. Chem. Soc. 27,1962, 3409
(10) The Peptides, Editors: E. Gross and J. Meienhofer; Academic Press, Vol. 1, 1979
(11) Andreu D., Albericio F., Solé N., Munson M., Ferrer M., Barany G., Methods in Molecular Biology: Peptide Synthesis Protocols; Vol. 35, p. 117:. Edited by: M.W. Pennington and B.M. Dunn, 1994 Humana Press Inc., Totowa, NJ

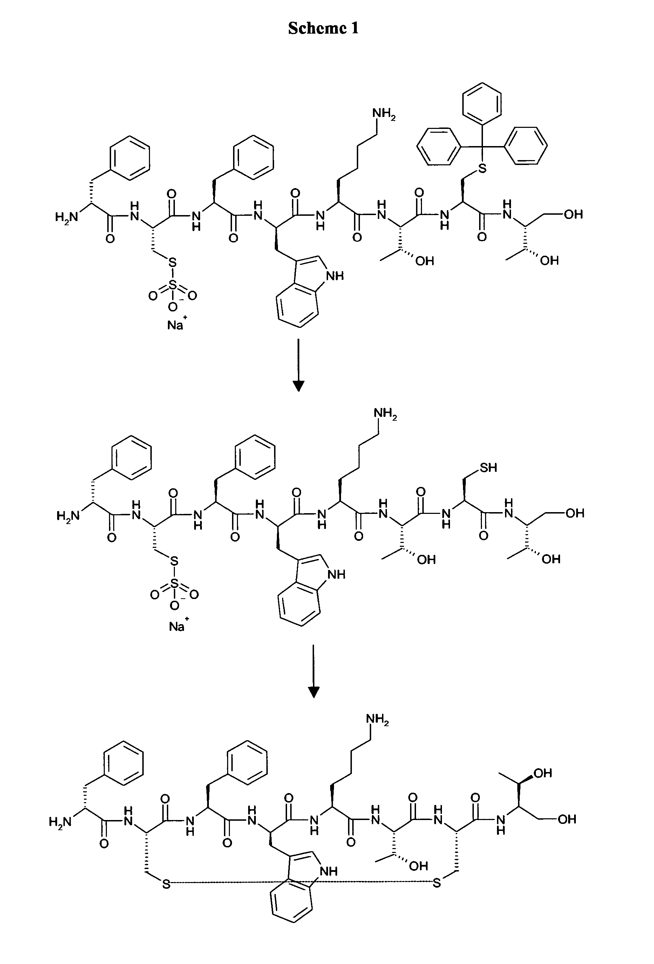

## Claims

1. A process for the cyclisation of peptide or peptidomimetic molecules comprising:
(a) preparation of a linear precursor containing an -S-sulfonate group and an -SH group protected with an acid-labile protective group;
(b) selective liberation of the -SH group protected with said acid-labile protective group in an anhydrous aprotic non-polar organic solvent in the presence of at least one carbocation scavenger at a pH of less than 3;
(c) dissolution and subsequent cyclisation of the partially deprotected linear precursor at a pH of between 5 and 9.

2. A process according to claim 1, **characterised in that** said acid-labile protective group is selected from among 4-methoxytrityl, S-2,4,6-trimethoxybenzyl, S-4,4',4"-trimethoxytrityl and trityl.

3. A process according to claim 2, **characterised in that** said protective group is trityl.

4. A process according to claims 1-3, **characterised in that** step (b) is performed in the presence of trifluoroacetic acid.

5. A process according to claims 1-4, **characterised in that** said carbocation scavenger is triisopropylsilane.

6. A process according to claims 1-5, **characterised in that** step (b) is performed in the presence of a mixture of trifluoroacetic acid and triisopropylsilane.

7. A process according to claim 6, **characterised in that** the trifluoroacetic acid and the triisopropylsilane are present ratios by volume of between 90:10 and 99:1.

8. A process according to claim 7, **characterised in that** the trifluoroacetic acid and the triisopropylsilane are present ratios by volume of approx. 95:5.

9. A process according to claim 6, **characterised in that** it is performed in the presence of anisole, tryptophan derivatives, such as tryptophan methyl ester, and/or phenol.

10. A process according to claim 9, **characterised in that** said anisole, tryptophan derivatives and/or phenol are present in quantities of between 1 and 3, preferably 2, equivalents, relative to the linear precursor.

11. A process according to claims 1-10, **characterised in that** the anhydrous aprotic non-polar organic solvent used in step (b) is selected from among dichloromethane, chloroform, dichloroethane, trichloroethylene and/or tetrachloroethylene, preferably dichloromethane.

12. A process according to claim 11, **characterised in that** said solvent is present in a quantity of between 25 and 10 litres, preferably between 20 and 15 litres, per mole of linear precursor.

13. A process according to claims 1-12, **characterised in that** step (c) is performed at a pH of between 7 and 9, preferably between 8 and 8.5.

14. A process according to claims 1-12, **characterised in that** the solvent of step (c) is a mixture of an aprotic polar organic solvent and an aqueous buffer.

15. A process according to claim 14, **characterised in that** said aprotic polar organic solvent is selected from among acetonitrile, tetrahydrofuran and/or acetone.

16. A process according to claim 14, **characterised in that** said aqueous buffer is a buffer based on sodium and/or potassium phosphate or on ammonium acetates.

17. A process according to claim 14, **characterised in that** said aprotic polar organic solvent is present in a quantity of 0.5-1.5 volumes per volume of said aqueous buffer.

18. A process according to claim 14, **characterised in that** said aprotic polar organic solvent is present in a quantity of 0.8-1.2 volumes per volume of said aqueous buffer.

19. A process according to claims 13-18, **characterised in that** it is performed at a temperature of between 0 and 50°C, preferably at a temperature of between 15 and 30°C.

20. A process according to claims 13-19, **characterised in that** it is performed for a period of between 5 minutes and 24 hours, preferably between 5 and 60 minutes.

## Patentansprüche

1. Verfahren zur Cyclisierung von Peptid- oder Peptid-nachahmenden Molekülen, das umfasst:
(a) Herstellung eines linearen Precursors, der eine S-Sulfonatgruppe und eine mit einer säureempfindlichen Schutzgruppe geschützten -SH Gruppe beinhaltet;
(b) selektive Freisetzung der mit der säureempfindlichen Schutzgruppe geschützten -SH Gruppe in einem wasserfreien, aprotischen, unpolaren organischen Lösungsmittel in der Gegenwart von mindestens einem Karbokationen-Fänger bei einem pH von weniger als 3;
(c) Lösen und anschließende Cyclisierung des partiell entschützten linearen Precursors bei einem pH zwischen 5 und 9.

2. Verfahren gemäß Anspruch 1, **dadurch gekennzeichnet, dass** die säureempfindliche Schutzgruppe unter 4-Methoxytrityl, S-2,4,6-Trimethoxybenzyl, S-4,4',4"-Trimethoxytrityl und Trityl ausgewählt wird.

3. Verfahren gemäß Anspruch 2, **dadurch gekennzeichnet, dass** die Schutzgruppe Trityl ist.

4. Verfahren gemäß Ansprüchen 1-3, **dadurch gekennzeichnet, dass** Stufe (b) in der Gegenwart von Trifluoressigsäure ausgeführt wird.

5. Verfahren gemäß Ansprüchen 1-4, **dadurch gekennzeichnet, dass** der Karbokationen-Fänger Triisopropylsilan ist.

6. Verfahren gemäß Ansprüchen 1-5, **dadurch gekennzeichnet, dass** die Stufe (b) in der Gegenwart einer Mischung aus Trifluoressigsäure und Triisopropylsilan ausgeführt wird.

7. Verfahren gemäß Anspruch 6, **dadurch gekennzeichnet, dass** die Trifluoressigsäure und das Triisopropylsilan in Volumenverhältnissen von zwischen 90:10 und 99:1 vorliegen.

8. Verfahren gemäß Anspruch 7, **dadurch gekennzeichnet, dass** die Trifluoressigsäure und das Triisopropylsilan in Volumenverhältnissen von ungefähr 95:5 vorliegen.

9. Verfahren gemäß Anspruch 6, **dadurch gekennzeichnet, dass** es in der Gegenwart von Anisol, Tryptophanderivaten wie Tryptophanmethylester, und/oder Phenol ausgeführt wird.

10. Verfahren gemäß Anspruch 9, **dadurch gekennzeichnet, dass** das Anisol, Tryptophanderivate und/oder Phenol in Mengen zwischen 1 und 3, vorzugsweise 2 Äquivalenten bezüglich des linearen Presursors vorliegen.

11. Verfahren gemäß Ansprüchen 1-10, **dadurch gekennzeichnet, dass** das wasserfreie, aprotische, unpolare organische Lösungsmittel, das in Stufe (b) verwendet wird, unter Dichlormethan, Chloroform, Dichlorethan, Trichlorethylen und/oder Tetrachlorethylen, vorzugsweise Dichlormethan, ausgewählt wird.

12. Verfahren gemäß Anspruch 11, **dadurch gekennzeichnet, dass** das Lösungsmittel in einer Menge von zwischen 25 und 10 Litern, vorzugsweise zwischen 20 und 15 Litern pro Mol linearen Precursors vorliegt.

13. Verfahren gemäß Ansprüchen 1-12, **dadurch gekennzeichnet, dass** Stufe (c) bei einem pH zwischen 7 und 9, vorzugsweise zwischen 8 und 8,5 ausgeführt wird.

14. Verfahren gemäß Ansprüchen 1-12, **dadurch gekennzeichnet, dass** das Lösungsmittel der Stufe (c) eine Mischung eines aprotischen, polaren organischen Lösungsmittels und eines wässrigen Puffers ist.

15. Verfahren gemäß Anspruch 14, **dadurch gekennzeichnet, dass** das aprotische, polare organische Lösungsmittel unter Acetonitril, Tetrahydrofuran und/oder Aceton ausgewählt wird.

16. Verfahren gemäß Anspruch 14, **dadurch gekennzeichnet, dass** der wässrige Puffer ein Puffer ist, der auf Natrium- und/oder Kaliumphosphat oder auf Ammoniumacetaten basiert.

17. Verfahren gemäß Anspruch 14, **dadurch gekennzeichnet, dass** das aprotische, polare organische Lösungsmittel in einer Menge von 0,5-1,5 Volumeneinheiten pro Volumen des wässrigen Puffers vorliegt.

18. Verfahren gemäß Anspruch 14, **dadurch gekennzeichnet, dass** das aprotische, polare organische Lösungsmittel in einer Menge von 0,8-1,2 Volumeneinheiten pro Volumen des wässrigen Puffers vorliegt.

19. Verfahren gemäß Ansprüchen 13-18, **dadurch gekennzeichnet, dass** es bei einer Temperatur zwischen 0 und 50 °C, vorzugsweise bei einer Temperatur zwischen 15 und 30 °C ausgeführt wird.

20. Verfahren gemäß Ansprüchen 13-19, **dadurch gekennzeichnet, dass** es für einen Zeitraum zwischen 5 Minuten und 24 Stunden, vorzugsweise zwischen 5 und 60 Minuten ausgeführt wird.

## Revendications

1. Procédé de cyclisation de molécules peptidiques ou peptidomimétiques comprenant :
(a) la préparation d'un précurseur linéaire contenant un groupe -S-sulfonate et un groupe -SH protégé par un groupe protecteur acido-sensible ;
(b) la libération sélective du groupe -SH protégé par ledit groupe protecteur acido-sensible dans un solvant organique non polaire aprotique anhydre en présence d'au moins un piège à carbocation à un pH inférieur à 3 ;
(c) la dissolution et la cyclisation ultérieure du précurseur linéaire partiellement déprotégé à un pH compris entre 5 et 9.

2. Procédé selon la revendication 1, **caractérisé en ce que** ledit groupe protecteur acido-sensible est sélectionné parmi les groupes 4-méthoxytrityle, S-2,4,6-triméthoxybenzyle, S-4,4',4''-triméthoxytrityle et trityle.

3. Procédé selon la revendication 2, **caractérisé en ce que** ledit groupe protecteur est un groupe trityle.

4. Procédé selon les revendications 1 à 3, **caractérisé en ce que** l'étape (b) est effectuée en présence d'acide trifluoroacétique.

5. Procédé selon les revendications 1 à 4, **caractérisé en ce que** ledit piège à carbocation est le triisopropylsilane.

6. Procédé selon les revendications 1 à 5, **caractérisé en ce que** l'étape (b) est effectuée en présence d'un mélange d'acide trifluoroacétique et de triisopropylsilane.

7. Procédé selon la revendication 6, **caractérisé en ce que** d'acide trifluoroacétique et le triisopropylsilane sont présents dans un rapport compris entre 90:10 et 99:1 en volume.

8. Procédé selon la revendication 7, **caractérisé en ce que** d'acide trifluoroacétique et le triisopropylsilane sont présents dans un rapport d'environ 95:5 en volume.

9. Procédé selon la revendication 6, **caractérisé en ce qu'**il est réalisé en présence de dérivés d'anisole, tryptophane, comme l'ester méthylique de tryptophane et/ou le phénol.

10. Procédé selon la revendication 9, **caractérisé en ce que** lesdits dérivés d'anisole, tryptophane et/ou phénol sont présents en quantité comprise entre 1 et 3, de préférence égale à 2, équivalents, par rapport au précurseur linéaire.

11. Procédé selon les revendications 1 à 10, **caractérisé en ce que** le solvant organique non polaire aprotique anhydre utilisé à l'étape (b) est choisi parmi le dichlorométhane, le chloroforme, le dichloroéthane, le trichloroéthylène et/ou le tétrachloroéthylène, et est de préférence le dichlorométhane.

12. Procédé selon la revendication 11, **caractérisé en ce que** ledit solvant est présent en quantité comprise entre 25 et 10 litres, de préférence entre 20 et 15 litres, par mole de précurseur linéaire.

13. Procédé selon les revendications 1 à 12, **caractérisé en ce que** l'étape (c) est effectuée à un pH compris entre 7 et 9, de préférence entre 8 et 8,5.

14. Procédé selon les revendications 1 à 12, **caractérisé en ce que** le solvant utilisé à l'étape (c) est un mélange d'un solvant organique polaire aprotique et d'un tampon aqueux.

15. Procédé selon la revendication 14, **caractérisé en ce que** ledit solvant organique polaire aprotique est choisi parmi l'acétonitrile, le tétrahydrofurane et/ou l'acétone.

16. Procédé selon la revendication 14, **caractérisé en ce que** ledit tampon aqueux est un tampon à base de sodium et/ou de phosphate de potassium ou à base d'acétates d'ammonium.

17. Procédé selon la revendication 14, **caractérisé en ce que** ledit solvant organique polaire aprotique est présent en quantité de 0,5 à 1,5 volumes par volume dudit tampon aqueux.

18. Procédé selon la revendication 14, **caractérisé en ce que** ledit solvant organique polaire aprotique est présent en quantité de 0,8 à 1,2 volumes par volume dudit tampon aqueux.

19. Procédé selon les revendications 13 à 18, **caractérisé en ce qu'**il est effectué à une température comprise entre 0 et 50°C, et de préférence à une température comprise entre 15 et 30°C.

20. Procédé selon les revendications 13 à 19, **caractérisé en ce qu'**il est effectué pendant une période comprise entre 5 minutes et 24 heures, de préférence entre 5 et 60 minutes.
